# EUROPEAN PATENT SPECIFICATION

(11) **EP 0 593 501 B1**
(45) Date of publication and mention of the grant of the patent: **20.03.2002**
(21) Application number: 92910944.5
(22) Date of filing: 03.06.1992
(51) Int. Cl.: C12N 15/05, C12N 15/29, C12N 15/82, A01H 5/00, C07K 14/415, A01N 65/00

(54) **BIOCIDAL PROTEINS**
BIOZIDE PROTEINE
PROTEINES BIOCIDES

(30) Priority: 07.06.1991 GB 91123000
(43) Date of publication of application: 27.04.1994
(73) Proprietor: Syngenta Limited, Haslemere, Surrey GU27 3JE (GB)
(72) Inventor: BROEKAERT, Willem, Frans, B-1700 Dilbeek (BE); CAMMUE, Bruno, Philippe, Angelo, B-1652 Alsemberg (BE); REES, Sarah, Bronwen 32 Micheldever Way, Bracknell Berkshire RG12 3XX (GB); VANDERLEYDEN, Jozef, B-3001 Heverlee (BE)
(74) Representative: Gaal, Jozsef Christopher
(86) International application number: GB9200999
(87) International publication number: WO9221699

(56) References cited:
- EP-A- 334 383
- EP-A- 354 687
- EP-A- 388 186
- Chemical Abstracts vol. 116, no. 21, issued 1992, May 25 (Columbus, Ohio, U.S.A.), W.F. BROEKAERT et al., "Anti- microbial peptides from Amaranthus caudatus seeds with sequence homology to the cysteine/glycine-rich domain of chitin-binding proteins", page 410, abstract no. 211129a, & Biochemistry 1992, 31(17), 4308-14 (Eng)
- Chemical Abstracts vol. 100, no. 9, issued 1984, February 27 (Columbus, Ohio, U.S.A.), A. BATRA et al., "Gas chromatography identification of fatty acid methyl esters and antimicrobial activity of Amaranthus hybridus(Amaranthaceae) oil", page 487, abstract no. 66856y, & Grasas Aceteis (Seville) 1983, 34(5) 329-31 (Eng)
- Chemical Abstracts vol. 88, no. 5, issued 30 January 1978 (Columbus, Ohio, U.S.A.), T. TANIGUCHI, "Anti-viral substances", page 78, abstract no. 32465p, & Shokubutsu Boeki 1977, 31(6) 219-24 (Japan)
- Chemical Abstracts vol. 112, no. 5, issued 29 January 1990 (Columbus, Ohio, U.S.A.), RINDERLE et al., "Isolation and characterization of amaranthin, a lectin present in the seeds of Amaranthus caudatus, that recognizes the T- (or cryptic T-) antigen", page 435, abstract no. 34252e, & J. Biol. Chem. 1989, 264(27), 16123-31 (Eng)

## Description

This invention relates to biocidal proteins, processes for their manufacture and use, and DNA sequences coding for them. In particular, it relates to antimicrobial proteins isolated from Amaranthus.

Amaranthus caudatus (amaranth) belongs to a large family, the Amaranthaceae, of herbs and shrubs which grow widely in tropical, sub-tropical and temperate regions. Amaranth is an ancient food crop of the Americas, and is still cultivated for grain production in parts of Central and South America, Asia and Africa. Amaranth seeds can be popped, toasted, cooked for gruel, milled into flour or made into flat breads, and have a particularly high nutritive value (Betschart et al, 1981, J Food Sci, 46, 1181-1187; Pedersen et al, 1987, Plant Foods Hum Nutr, 36, 309-324). Amaranth is also cultivated world-wide as a garden ornamental.

Although plants normally grow on substrates that are extremely rich in fungal organisms, infection remains a rare event. To keep out potential invaders, plants produce a wide array of antifungal compounds, either in a constitutive or an inducible manner. The best studied of these are phytoalexins, secondary metabolites with a broad antimicrobial activity spectrum that are specifically synthesised upon perception of appropriate defence-related signal molecules. The production of phytoalexins depends on the transcriptional activation of a series of genes encoding enzymes of the phytoalexin biosynthetic pathway. During the last decade, however, it has become increasingly clear that some plant proteins can play a more direct role in the control of phytopathogenic fungi. Several classes of proteins with antifungal properties have now been identified, including beta-1,3-glucanases, ribosome-inactivating proteins, thionins, chitinases, chitin-binding lectins and zeamatins.

Chitin (poly-β-1,4-N-acetyl-D-glucosamine) is a polysaccharide occurring in the cell wall of fungi and in the exoskeleton of invertebrates. Although plants do not contain chitin or chitin-like structures, proteins exhibiting strong affinity to this polysaccharide have been isolated from different plant sources (Raikhel and Broekaert, 1991, in: Verma, ed, Control of plant gene expression, in press).

Examples of such chitin-binding proteins are basic chitinases from bean (Boller et al, 1983, Planta, 157:22-31), wheat (Molano et al, 1979, J Biol Chem, 254:4901-4907) and tobacco (Shinshi et al, 1987, Proc Natl Acad Sci USA, 84:89-93); chitin-binding lectins from wheat (Rice and Etzler, 1974, Biochem Biophys Res Comm, 59:414-419), barley (Peumans et al, 1982, Biochem J, 203:239-143), rice (Tsuda, 1979, J Biochem, 86:1451-1461), and stinging nettle (Peumans,et al, 1983, FEBS Lets, 177:99-103); and a small protein from rubber tree latex called hevein (Van Parijs et al, 1991, Planta, 183:258-264). All these chitin-binding proteins share a homologous cysteine/glycine-rich domain of about 40-43 amino acids, which is either repeated two-fold (in the nettle lectin), four-fold (in wheat, barley and rice lectins) or fused to an unrelated domain (in basic chitinases).

The exact physiological role of these proteins remains uncertain, but they all show antibiotic activity in vitro, suggesting a defence-related function. Antifungal properties have been ascribed to chitinases (Schlumbaum et al, 1986, Nature, 324:365-367; Broekaert et al, 1988, Physiol Mol Plant Pathol, 33:319-331), nettle lectin (Broekaert et al, 1989, Science, 245:1100-1102) and hevein (Van Parijs et al, 1991, Planta, 183:258-264). The wheat lectin causes deleterious effects on the development of insect larvae (Murdock et al, 1990, Phytochem, 29:85-89; Czapla and Lang, 1990, J Econ Entomol, 83:2480-2485). However, it is not established whether or not the observed antibiotic effects on fungi or insects are related to the chitin-binding activity of these proteins.

Successful application of chitin-binding proteins, especially chitinases, in the protection of plants against fungal disease has been reported. In US Patent Number 4940840 (DNA Plant Technology Corporation), tobacco plants expressing a chitinase gene from the bacterium Serratia marcescens appear to be less sensitive to the fungus Alternaria longipes. European Patent Application Number 418695 (Ciba Geigy) describes the use of regulatory DNA sequences from tobacco chitinase gene to drive expression of introduced genes producing transgenic plants with improved resistance to pathogens. Patent Application Number WO9007001 (Du Pont de Nemours Company) describes production of transgenic plants which over-express a chitinase gene giving improved resistance to fungal pathogens.

We have now purified a new class of potent antimicrobial proteins which exhibit chitin-binding properties.

According to the present invention there is provided an isolated anti-microbial protein which is a member of a class of proteins having an amino acid sequence containing the cysteine/glycine domain of chitin-binding proteins derived from plants characterised in that said anti-microbial protein is capable of being isolated from *Amaranthus* seeds or seeds of a closely related species and has: (a) a higher ratio of basic amino acids to acidic amino acids; and (b) an additional amino-terminal residue; and (c) the occurrence of a gap of four amino acids at position 6 to 9; and (d) the lack of a carboxyl terminal portion of 10-12 residues, when compared to the regular 40-43 amino acid cysteine/glycene-rich domain found in said chitin-binding proteins.

In further aspects, this invention comprises a vector containing a DNA sequence coding for a protein according to the invention. The DNA may be cloned or transformed into a biological system allowing expression of the encoded protein.

The invention also comprises plants transformed with recombinant DNA encoding an antimicrobial protein according to the invention.

The invention also comprises a process of combating fungi or bacteria whereby they are exposed to the proteins according to the invention wherein the process is not an *in vivo* one.

A new class of potent antimicrobial proteins has been isolated from seeds of *Amaranthus caudatus* (amaranth). The class includes two protein factors, hereafter called Ac-AMP1 *(Amaranthus caudatus -* Antimicrobial Protein 1) and Ac-AMP2 *(Amaranthus caudatus -* Antimicrobial Protein 2) respectively. Both are dimeric proteins, composed of two identical 3 kDa subunits. Both proteins are highly basic and have pI values above 10. Proteins with similar antifungal activity have been extracted from the seed of several closely related species, including *Amaranthus paniculatus, Amaranthus retroflexus, Amaranthus lividus* and *Gomphrena globossa.*

The amino acid sequence of Ac-AMP1 (29 residues) is identical to that of Ac-AMP2 (30 residues), except that the latter has one additional residue at the carboxyl- terminus. Knowledge of these sequences enables manufacture of the Ac-AMP proteins using a standard peptide synthesiser.

The amino acid sequences of Ac-AMP1 and Ac-AMP2 are highly homologous to the cysteine/glycine-rich domain occurring in many chitin-binding proteins. Moreover, Ac-AMP1 and Ac-AMP2 bind to chitin and can be desorbed at low pH (a property shared by chitinases and lectins), and so can be considered as new members of the family of chitin-binding proteins. However, when compared to the regular 40-43 amino acid cysteine/glycine-rich domains found in the chitin-binding proteins previously characterised, the Ac-AMPs distinguish themselves by several features. These include a higher abundance of basic amino acids, the presence of an additional amino-terminal residue, the occurrence of a gap of four amino acids at position 6 to 9, and the lack of a carboxyl-terminal portion of 10-12 residues.

cDNA encoding the Ac-AMPs has been isolated and sequenced. The cDNA encoding Ac-AMP2 has been identified. It encodes an 86-amino acid preprotein and a 25-amino acid carboxy-terminal extension. The structure of this preprotein differs from all other known precursors of chitin-binding proteins. The cDNA encoding Ac-AMP1 has been identified as a post-translational cleavage product of Ac-AMP2.

This DNA can be manufactured using a standard nucleic acid synthesiser, or suitable probes (derived from the known sequence) can be used to isolate the actual Ac-AMP gene(s) and control sequences from the plant genome. This genetic material can then be cloned into a biological system, excluding a human or animal body, which allows expression of the proteins under the control of a constitutive or inducible promoter. Hence the proteins can be produced in a suitable micro-organism or cultured cell, extracted and isolated for use. Suitable micro-organisms include Escherichia coli and Pseudomonas. Suitable cells include cultured insect cells and cultured mammalian cells. The DNA can also be transformed by known methods into any plant species, so that the antimicrobial proteins are expressed within the plant.

Plant cells according to the invention may be transformed with constructs of the invention according to a variety of known methods (Agrobacterium Ti plasmids, electroporation, microinjection, microprojectile gun, etc). The transformed cells may then in suitable cases be regenerated into whole plants in which the new nuclear material is stably incorporated into the genome. Both transformed monocot and dicot plants may be obtained in this way, although the latter are usually more easy to regenerate.

Examples of genetically modified plants according to the present invention include: fruit such as tomatoes, mangoes, peaches, apples, pears, strawberries, bananas and melons; field crops such as canola, sunflower, tobacco, sugarbeet, small-grain cereals such as wheat, barley and rice, maize and cotton: and vegetables such as carrot, lettuce, cabbage and onion.

The Ac-AMP proteins show a wide range of antifungal activity, and are also active against gram-positive bacteria. The proteins could be used as fungicides or antibiotics by application to plant parts. The proteins could also be used to combat fungal or bacterial disease by expression within plant bodies.

Both Ac-AMP1 and Ac-AMP2 show surprisingly high activity: they inhibit the growth of a variety of plant pathogenic fungi at much lower doses than previously-known antifungal chitin-binding proteins. The antifungal effect of the novel proteins is antagonised by Ca²⁺.

Some chitin-binding proteins are known to have an effect against insects which possess an exoskeleton comprising chitin. The sequence similarity between the Ac-AMPs and known chitin-binding proteins implies that the Ac-AMPs may also possess insecticidal properties.

The Ac-AMP proteins can be isolated and purified from Amaranthus caudatus seeds, synthesised artificially from their known amino acid sequence, or produced within a suitable micro-organism by expression of recombinant DNA. The proteins may also be expressed within a transgenic plant.

The invention may be further understood by reference to the drawings, in which:
Figure 1 shows the cation exchange chromatogram for the antifungal proteins and the associated graph of fungal growth inhibition.
Figure 2A shows the HPLC profile of purified Ac-AMP1.
Figure 2B shows the HPLC profile of purified Ac-AMP2.
Figure 3 shows the SDS-PAGE analysis of the purified antifungal proteins.
Figure 4A shows the amino acid sequences of Ac-AMP1 and Ac-AMP2.
Figure 4B shows the alignment of amino acid sequences from tobacco chitinase, bean chitinase, hevein, wheat lectin, nettle lectin, and Ac-AMP2.
Figure 5 shows the SDS-PAGE analysis of different eluates after affinity chromatography of antifungal proteins on chitin.
Figure 6 shows the growth inhibition curves of fungi measured at varying concentrations of antifungal proteins.
Figure 7 shows the growth inhibition curves of B cinerea measured at varying concentrations of antifungal proteins with and without different additions of KCl or CaCl₂.
Figure 8 shows the nucleotide sequence and deduced amino acid sequence of a cDNA clone encoding Ac-AMP2.
Figure 9 shows the structure of the expression vectors pAC11 and pAC12.
Figure 10 shows the structure of the plant transformation vectors pAC111 and pAC112.
The following Examples illustrate the invention.

### EXAMPLE 1

### Extraction of basic heat-stable proteins from Amaranthus caudatus seeds.

Ammonium sulphate fractionation of proteins precipitating in the interval of 30 to 75% relative saturation was followed by isolation of the basic protein fraction (pI>9) by passage over a Q-Sepharose (Pharmacia) anion exchange column equilibrated at pH 9. The detailed methods are described below.

One kg of A caudatus seeds (obtained from Gonthier, Wanze, Belgium) was ground in a coffee mill and the resulting meal was extracted for 2 hours at 4°C with 3 litres of an ice-cold extraction buffer containing 10 mM NaH₂PO₄, 15 mM Na₂HPO₄, 100 mM KCl, 2 mM EDTA, 2 mM thiourea, 1 mM PMSF and 1 mg/l leupeptin. The homogenate was squeezed through cheesecloth and clarified by centrifugation (5 min at 7,000 x g). Solid ammonium sulphate was added to the supernatant to obtain 30% relative saturation and the precipitate formed after standing for 1 hour at room temperature was removed by centrifugation (10 min at 7,000 x g). The supernatant was adjusted to 75% relative ammonium sulphate saturation and the precipitate formed overnight at room temperature collected by centrifugation (30 min at 7,000 x g). After redissolving the pellet in 300 ml distilled water, the insoluble material was removed by further centrifugation (20 min at 7,000 x g). The clear supernatant was dialyzed extensively against distilled water using benzoylated cellulose tubing (Sigma, St Louis, MO) with a molecular weight cut off of 2,000 Da. After dialysis the solution was adjusted to 50 mM Tris-HCl (pH 9) by addition of the ten-fold concentrated buffer, and subsequently passed over a Q-Sepharose Fast Flow (Pharmacia, Uppsala, Sweden) column (12 x 5 cm) in equilibrium with 50 mM Tris-HCl (pH 9). The proteins passed through the column were dialyzed extensively against 20 mM sodium phosphate buffer (pH 7).

This material represents the basic protein fraction of A caudatus seeds. Its further chromatographic purification is described in Example 3.

### EXAMPLE 2

### Antifungal and antibacterial activity assays.

Antifungal activity was measured by microspectrophotometry as previously described (Broekaert, 1990, FEMS Microbiol Lett, 69:55-60). Routinely, tests were performed with 20 µl of a (filter-sterilized) test solution and 80 µl of a fungal spore suspension (2 x 10⁴ spores/ml) or mycelium fragments in half strength Potato Dextrose Broth (Difco). For experiments on the antagonistic effect of cations, a synthetic growth medium was used instead of Potato Dextrose Broth. The synthetic growth medium consisted of K₂HPO₄ (2.5 mM), MgSO₄ (50 µM), CaCl₂ (50 µM), FeSO₄ (5 µM), CoCl₂ (0.1 µM), CuSO₄ (0.1 µM), Na₂MoO₄ (2 µM), H₃BO₃ (0.5 µM), KI (0.1 µM) , ZnSO₄ (0.5 µM), MnSO₄ (0.1 µM), glucose (10 g/l), asparagine (1 g/l), methionine (20 mg/l), myo-inositol (2 mg/l), biotin (0.2 mg/l), thiamine-HCl (1 mg/l), and pyridoxine-HCl (0.2 mg/l). Control microcultures contained 20 µl of sterile distilled water and 80 µl of the fungal spore suspension.

Unless otherwise stated the test organism was Fusarium culmorum (strain IMI 180420) and incubation was carried out at 25°C for 48 hours. Percent growth inhibition is defined as 100 times the ratio of the corrected absorbance of the control microculture minus the corrected absorbance of the test microculture over the corrected absorbance at 595 nm of the control microculture. The corrected absorbance values equal the absorbance at 595 nm of the culture measured after 48 hours minus the absorbance at 595 nm measured after 30 min. Values of growth inhibition lower than 15% are not indicated on the chromatograms. The antifungal activity (units per ml) is calculated as 50 times the dilution factor of a test solution at which 50% growth inhibition is obtained under the given assay conditions.

Antibacterial activity was measured microspectrophotometrically as follows. A bacterial suspension was prepared by inoculating soft nutrient agarose (tryptone, 10 g/l; Seaplaque agarose (FMC), 5 g/l) and kept at 37°C to prevent solidification. Aliquots (80 µl) of the bacterial suspension (10⁵ colony forming units per ml) were added to filter-sterilized samples (20 µl) in flat-bottom 96-well microplates. The absorbance at 595 nm of the culture was measured with the aid of a microplate reader after 30 minutes and 24 hours of incubation at 28°C. Percent growth inhibition was calculated as described above for the antifungal activity assay.

### EXAMPLE 3

### Purification of antifungal proteins from A caudatus seeds.

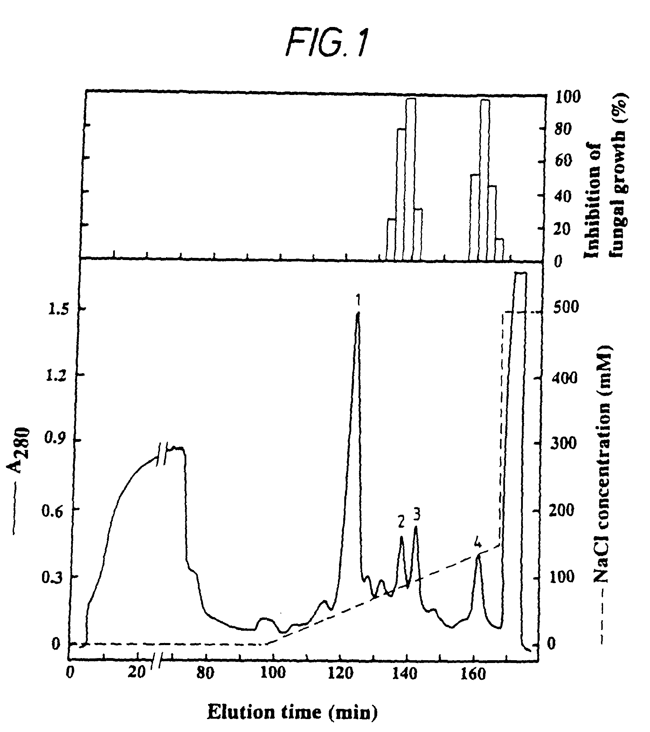

The starting material for the isolation of the A caudatus antifungal proteins was the basic protein fraction extracted from the mature seeds as in Example 1. These proteins were further separated by cation exchange chromatography, as shown in Figure 1.

About 100 mg of the basic protein fraction dissolved in 20 mM sodium phosphate buffer (pH 7) was applied on a S-Sepharose High Performance (Pharmacia) column (10 x 1.6 cm) previously equilibrated with the sodium phosphate buffer. The column was eluted at 3 ml\min with a linear gradient of 210 ml from 0 to 150 mM NaCl in 20 mM sodium phosphate buffer (pH 7). The eluate was monitored for protein by online measurement of the absorbance at 280 nm (results shown in the lower panel of Figure 1) and collected in 7.5 ml fractions of which 20 µl was tested in the microspectrophotometric antifungal activity assay described in Example 2 (results shown in the upper panel of Figure 1).

Upon fractionation, the mixture resolved into four distinct peaks (Figure 1). The antifungal activity co-eluted with the material from peaks 2 and 4, respectively.

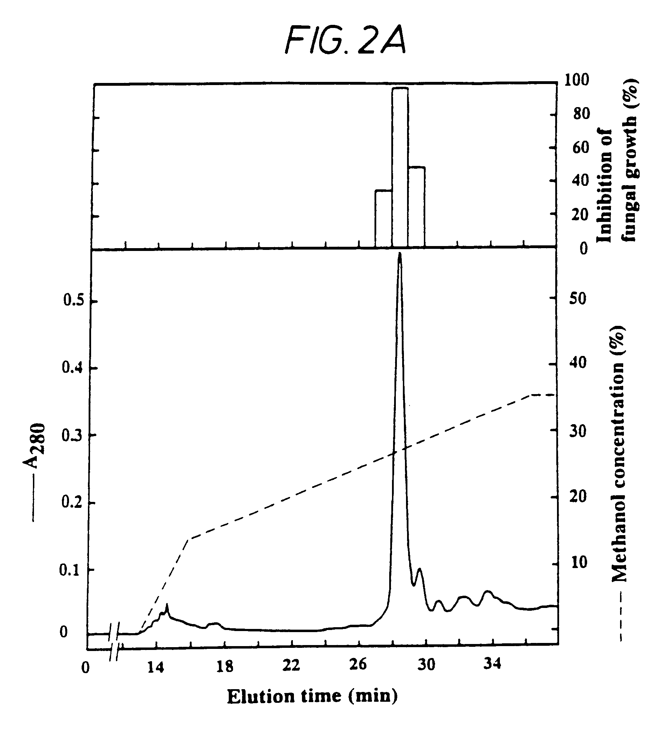

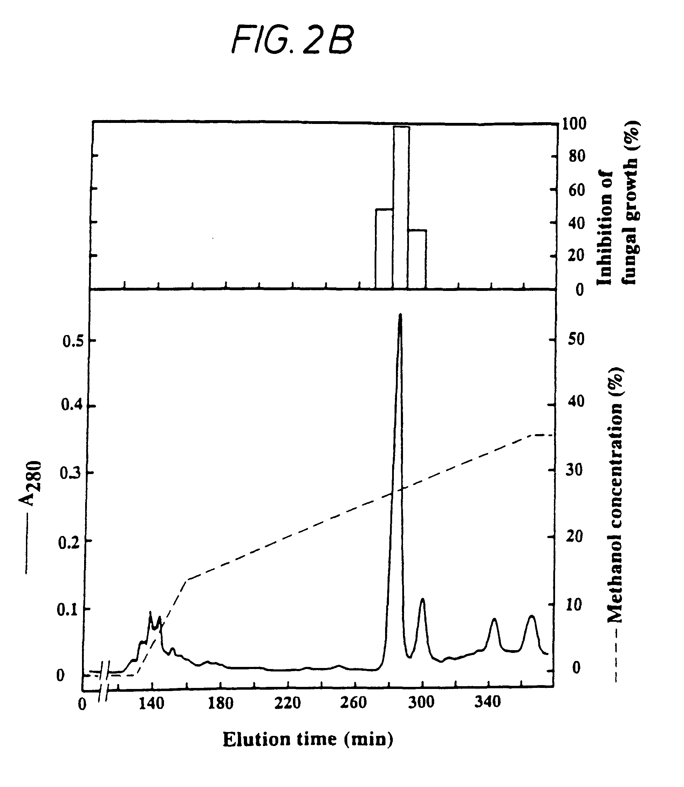

The active fractions were finally purified by reversed-phase chromatography. About 1 mg amounts of peak 2 material (Figure 2A) and peak 4 material (Figure 2B) were loaded on a Pep-S (porous silica C₂/C₁₈, Pharmacia) column (25 x 0.93 cm) in equilibrium with 0.1% TFA. The column was eluted at 5 ml/min with the following gradients (solvent B is methanol containing 0.1 % TFA): 0-3 min, 0-15% B; 3-23 min, 15-35% B; 23-25 min, 35-100% B. The eluate was monitored for protein by online measurement of the absorption at 280 nm. Five ml fractions of the eluate were collected, vacuum-dried, and finally dissolved in 0.5 ml distilled water of which 10 µl was used in a microspectrophotometric antifungal activity assay.

Figure 2A and Figure 2B show the HPLC profiles of purified peak 2 and peak 4 material respectively. The lower panels show monitoring of the eluate for protein by measurement of the absorption at 280 nm. Results of the microspectrophotometric antifungal activity assay are shown in the upper panels.

Both material from peak 2 and from peak 4 yielded well resolved major peaks that co-eluted with the antifungal activity. The active factor purified from peak 2 is called AC-AMP1 (Amaranthus caudatus antifungal protein 1), and that from peak 4 is designated analogously as Ac-AMP2.

### EXAMPLE 4

### Molecular structure of the purified antifungal proteins.

The molecular structure of the Ac-AMPs was further analysed. Sodium dodecyl sulphate polyacrylamide gel electrophoresis (SDS-PAGE) was performed on precast commercial gels (PhastGel High Density from Pharmacia) using a PhastSystem (Pharmacia) electrophoresis apparatus. The sample buffer contained 200 mM Tris-HCl (pH 8.3), 1% (w/v) SDS, 1 mM EDTA, 0.005% bromophenol blue and, unless otherwise stated, 1% (w/v) dithiothreitol (DTT). Proteins were fixed after electrophoresis in 6% glutaraldehyde and silver-stained according to Heukeshoven and Dernick (1985, Electrophoresis, 6:103-112).

The amaranth antifungal proteins were analysed by SDS-PAGE before and after reduction with dithiothreitol. Reduced Ac-AMP1 and Ac-AMP2 both migrated as single bands with an apparent molecular weight of about 3 kDa. However, in their unreduced state, Ac-AMP1 and Ac-AMP2 yielded a 4 kDa and a 6 kDa band respectively. It appears therefore that the antifungal factors are dimeric proteins stabilised by disulphide bridges, each comprised of two identical 3 kDa subunits. Attempts to determine the molecular weight of the native Ac-AMPs by gel filtration on either Superose-12 or Superdex-75 (Pharmacia) were unsuccessful as the proteins were retarded.

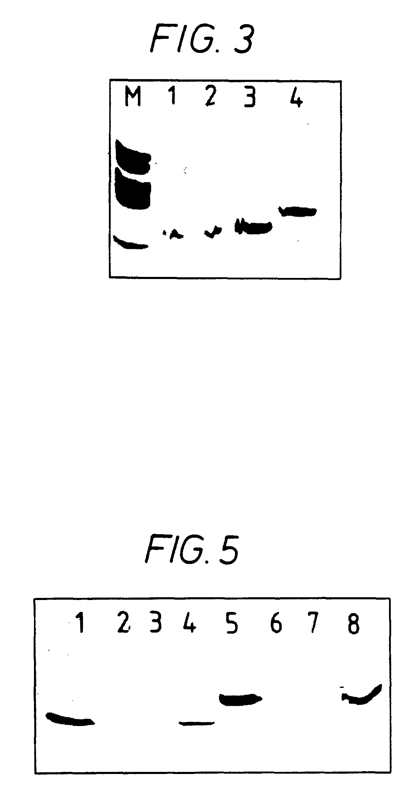

Figure 3 shows the SDS-PAGE analysis of the purified antifungal proteins: lane 1 is reduced Ac-AMP1, lane 2 is reduced Ac-AMP2, lane 3 is unreduced Ac-AMP1, lane 4 is unreduced Ac-AMP2. Two hundred nanograms of the proteins were separated on the gels. Lane M shows myoglobin fragments used as molecular weight markers (Pharmacia) with the following sizes: 17 kDa, 14.5 kDa, 8 kDa, 6 kDa, and 2.5 kDa.

Free cysteine thiol groups were assessed qualitatively as follows. Hundred µg amounts of reduced or unreduced proteins were dissolved in 6 M guanidinium-Cl containing 100 mM sodium phosphate buffer (pH 7) and 1 mM EDTA. The mixtures were allowed to react with 5,5'-dithionitrobenzoic acid and monitored for release of nitrothiobenzoate as described by Creighton (1989, Protein structure, a practical approach, 155-167). Reduction of the proteins was done by addition of Tris-HCl (pH 8.6) to 100 mM and dithiothreitol to 30 mM, followed by incubation at 45°C for 1 hour. The proteins were separated from the excess reagents by reversed-phase chromatography on a C₂/C₁₈ silica column.

The unreduced Ac-AMPs did not contain free cysteine thiol groups, whereas the reduced proteins did, indicating that all cysteine residues participate in disulphide bonds. The presence of a relatively high number of disulphide linkages in such small polypeptides suggests that the Ac-AMPs have compact structures.

The pI values of Ac-AMP1 and Ac-AMP2 were determined by isoelectric focusing and found to be 10.3 and over 10.6 respectively. Isoelectric focusing was performed on precast Immobiline Dry Strips (Pharmacia) rehydrated in 8 M urea, using marker proteins in the pI range from 4.7 to 10.6 (Pharmacia).

### EXAMPLE 5

### Amino acid sequencing of the Ac-AMPs.

Cysteine residues of the antifungal proteins were modified by S-carboxyamidomethylation as follows: 100 µg amounts of purified proteins were dissolved in 150 µl 0.3 M Tris-HCl (pH 8.6) containing 30 mM DTT and reacted for 1 hour at 45°C. Iodoacetamide was added to a final concentration of 100 mM and the mixture was kept in the dark at 37°C for 1 hour. The reaction was finally quenched by addition of DTT to a final concentration of 100 mM and allowed to react for an additional hour at 37°C. Removal of excess reagents was done by reversed-phase chromatography. The resulting protein fractions were subjected to amino acid sequence analysis in a 477A Protein Sequencer (Applied Biosystems) with on-line detection of phenylthiohydantoin amino acid derivatives in a 120A Analyser (Applied Biosystems).

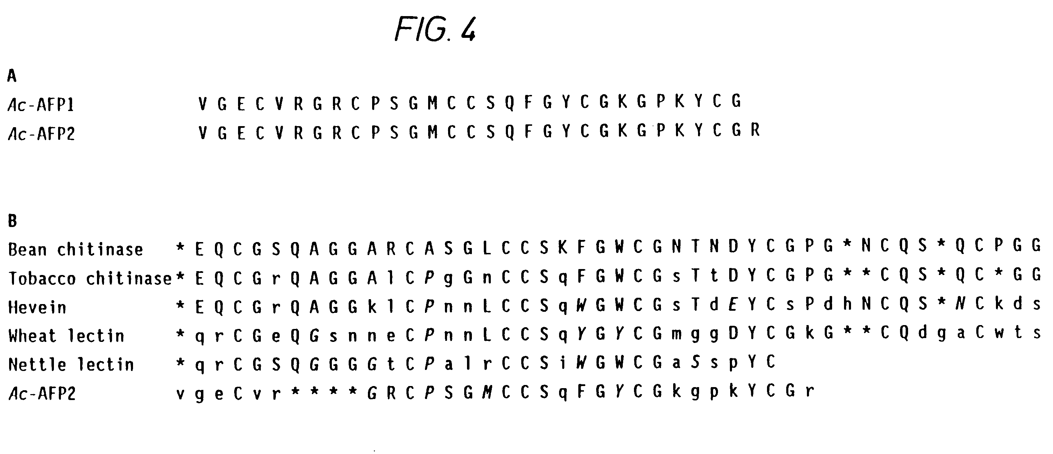

The amino acid sequence of the reduced and carboxyamidomethylated antifungal proteins was determined by direct N-terminal sequencing. Figure 4A shows the N-terminal amino acid sequences of AC-AMP1 and Ac-AMP2. AC-AMP1 is 29 amino acids in length, whereas Ac-AMP2 has 30 residues. The sequence of Ac-AMP2 is identical to that of Ac-AMP1 except that it has one additional amino acid at its carboxyl terminus (arginine). The Ac-AMPs are particularly rich in cysteine (6 residues), glycine (6 residues) and basic amino acids (4 and 5 residues for Ac-AMP1 and Ac-AMP2 respectively).

Ac-AMP1 appears to be a truncated form of Ac-AMP2. It is possible that the two proteins result from the same precursor molecule by differential post-translational processing.

The theoretical isoelectric points calculated from the sequence data are 10.1 and 11.0 for Ac-AMP1 and Ac-AMP2 respectively, assuming that all cysteine residues participate in disulphide linkages. These compare well to the measured pI values given in Example 4.

Figure 4B shows the alignment of N-terminal amino acid sequences from tobacco chitinase (Shinshi et al, 1987, Proc Natl Acad Sci USA, 84:89-93), bean chitinase (Broglie et al, 1986, Proc Natl Acad Sci USA, 83:6820-6824), hevein (Broekaert et al, 1990, Proc Natl Acad Sci USA, 87:7633-7637), wheat lectin (Raikhel and Wilkins, 1987, Proc Natl Acad Sci USA, 84:6745-6749), nettle lectin (Chapot et al, 1986, FEBS Lett, 195:231-234) and the sequence of Ac-AMP2. Sequence identities with the tobacco chitinase are indicated in capitals, conserved changes are marked in italics and non-conserved changes in lower case. Conserved changes are considered as substitutions within the amino acid homology groups FWY, MILV, RKH, ED, NQ, ST and PAG. Gaps introduced for optimal alignment are represented by asterisks.

The amino acid sequence of the Ac-AMPs shows striking similarity to the cysteine/glycine-rich domains of chitin-binding proteins, such as chitinases, chitin-binding lectins, and hevein. However, the Ac-AMPs also contain unique features. Sequence alignment of Ac-AMP2 and the N-terminus of a basic chitinase from tobacco (Figure 4B) showed 14 identical amino acids and 5 conserved changes in the first 30 residues. A single gap of four amino acids had to be introduced in the N-terminal portion of Ac-AMP2 to allow optimal alignment with the chitin-binding proteins. After introduction of this gap, all of the cysteine residues appeared at invariant positions.

### EXAMPLE 6

### Chitin-binding activity of the Ac-AMPs.

Because of the similarity at the amino acid sequence level between the Ac-AMPs and chitin-binding proteins, the ability of the amaranth antifungal proteins to bind on a chitin substrate was investigated.

Micro-columns packed with chitin were loaded with either Ac-AMP1 or Ac-AMP2 and subsequently eluted at neutral pH and low pH (pH 2.8). Chitin was prepared by N-acetylation of chitosan (Sigma, St Louis, MO) by the method of Molano et al (1977, Anal Biochem, 83:648-656). Protein samples (50 µg) dissolved in 1 ml phosphate buffered saline (pH 7) were applied on the chitin micro-column (2.5 x 6 mm) and recycled three times over the column. The column was eluted five times with 1 ml phosphate buffered saline (PBS) and once with 1 ml 100 mM acetic acid (pH 2.8). Fractions (1 ml) of the eluate were desalted and concentrated by reversed-phase chromatography and finally redissolved in 50 µl sample buffer for SDS-PAGE analysis.

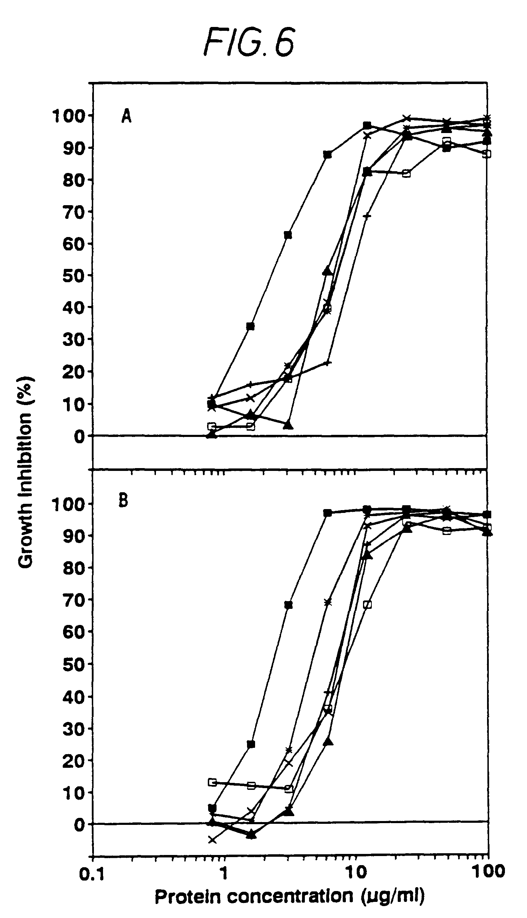

Figure 5 shows results of the SDS-PAGE analysis after this affinity chromatography of Ac-AMP1 (lanes 1-4) and Ac-AMP2 (lanes 5-8). Lanes 1 and 5 are the antifungal proteins at equivalent amounts as those loaded on the columns; lanes 2 and 6 are the fractions passed through the column; lanes 3 and 7 are the fractions eluted with PBS (pH 7); lanes 4 and 8 are the fractions eluted with 100 mM acetic acid (pH 2.8). It can be seen that the Ac-AMPs were absent from the fraction passed through the column and from the neutral pH washings, but instead were recovered in the low pH desorption buffer. These results indicate that both Ac-AMP1 and Ac-AMP2 exhibit affinity toward chitin.

### EXAMPLE 7

### Stability of the antifungal activity of the Ac-AMPs.

Tests for antifungal activity were performed with 20 µl samples diluted five-fold with growth medium containing Fusarium culmorum spores, according to the assay method given in Example 2. Untreated control samples consisted of the test proteins at 500 µg/ml in 10 mM sodium phosphate buffer (pH 7). For digestions, different proteases were added at 100 µg/ml and incubated at 37°C for 16 hours. Heat stability tests were performed by heating aliquots of the test proteins for 10 minutes at different temperatures up to 100°C. pH stability was tested by incubation of test proteins for 1 hour in either 20 mM glycine-HCl (pH 2) or glycine-NaOH (pH 11) and subsequent dialysis for 16 hours against 10 mM sodium phosphate buffer (pH 7) using benzoylated cellulose tubing. Reduction was done by addition of dithiothreitol at 30 mM and Tris-HCl (pH 8.6) at 300 mM. The reagents were removed by reversed-phase chromatography. Any of the control treatments containing only the reagents proved negative for antifungal activity after the dialysis or reversed-phase chromatography steps.

The antifungal activity of the Ac-AMPs was resistant to digestion by proteinase K, pronase E, chymotrypsin or trypsin. Moreover, the Ac-AMPs were not affected by heat treatments at up to 100°C for 10 minutes nor by exposure to pH conditions as extreme as pH 2 or pH 11. Reduction of their cysteine residues by dithiothreitol, however, completely abolished the antifungal activity.

The proteins are remarkably stable, since their biological activity is unaffected by protease treatments or by exposure to extreme temperatures and pH conditions. This stability may be due to a compact globular structure maintained by the relatively high number of disulphide linkages. These disulphide linkages are essential for biological activity.

### EXAMPLE 8

### Antifungal potency of the Ac-AMPs.

The antifungal potency of the Ac-AMPs was assessed on fourteen different plant pathogenic fungi, using the assay described in Example 2. Growth of fungi, collection and harvest of fungal spores, and preparation of mycelial fragments were done as previously described (Broekaert et al, 1990, FEMS Microbiol Lett, 69:55-60). The following fungal strains were used: Alternaria brassicola MUCL 20297, Ascochyta pisi MUCL 30164, Botrytis cinerea MUCL 30158, Cercospora beticola strain K897, Colletotrichum lindemuthianum MUCL 9577, Fusarium culmorum IMI 180420, Mycosphaerella fijiensis var fijiensis IMI 105378, Phytophthora infestans, Rhizoctonia solani CBS 207-84, Sclerotinia sclerotianum MUCL 30163, Septoria nodorum MUCL 30111, Trichoderma hamatum MUCL 29736, Verticillium dahliae MUCL 19210, and Venturia inaequalis MUCL 15927.

For C beticola, R solani, S sclerotianum, S nodorum, M fijiensis and P infestans, mycelial fragments were used as inoculum. All other fungi were inoculated as spores.

Figure 6 shows the dose-response curves of fungal growth inhibition measured at varying concentrations of Ac-AMP1 (panel A) and Ac-AMP2 (panel B) using the following test fungi: A brassicola (*); A pisi (x); B cinerea (+); C lindemuthianum (□); F culmorum (■); V dahliae(▲).

The antifungal activity of the Ac-AMPs on the fourteen plant pathogenic fungi listed above was compared to that of two known chitin-binding proteins, nettle lectin and pea chitinase. Table 1 summarises the results: IC₅₀ is the concentration (µg/ml) required for 50% growth inhibition after 48 hours of incubation. The IC₅₀ values for the slow growing fungi S nodorum and V inaequalis were measured after 5 and 15 days of incubation respectively. The nettle lectin (or Urtica dioica agglutinin, UDA) was isolated from stinging nettle (Urtica dioica) rhizomes as previously described (Peumans et al, 1983, FEBS Lett, 177:99-103). Chitinase was isolated from pea pods by the method of Mauch et al (1988, Plant Physiol, 87:325-333).

**TABLE 1**

| Antifungal activity of Ac-AMPs, nettle lectin and pea chitinase | | | | |
|---|---|---|---|---|
| **Fungus** | **IC**_{**50**} (µg/ml) | | | |
| | **Ac-AMP1** | **Ac-AMP2** | **UDA** | **chitinase** |
| A brassicola | 7 | 4 | 200 | 400 |
| A pisi | 8 | 8 | 1000 | >500 |
| B cinerea | 10 | 8 | >1000 | >500 |
| C beticola | 0.8 | 0.8 | ND | ND |
| C lindemuthianum | 8 | 8 | 20 | >500 |
| F culmorum | 2 | 2 | >1000 | >500 |
| M fijiensis | 3 | ND | 4 | ND |
| P infestans | 12 | ND | 4 | ND |
| R solani | 30 | 20 | 30 | ND |
| S sclerotianum | 20 | 10 | ND | ND |
| S nodorum | 20 | 20 | ND | ND |
| T hamatum | 7 | 3 | 90 | 1.5 |
| V dahliae | 6 | 5 | 80 | 500 |
| V inaequalis | ND | 3 | 1000 | ND |
| ND = not determined | | | | |

The concentration of Ac-AMP protein required for 50% growth inhibition after 48 hours of incubation (IC₅₀) varied from 0.8 to 30 µg/ml, depending on the test organism. The antifungal potency of Ac-AMP1 was almost identical to that of Ac-AMP2.

The Ac-AMPs are potent inhibitors of all fourteen fungi tested in this study. Their specificity is comparable to that of wheat thionin which also typically inhibits fungal growth with IC₅₀ values between 1 and 10 µg/ml (Cammue et al, 1992, J Biol Chem, 267, 2228-2233). Relative to other chitin-binding proteins, such as the nettle lectin or chitinase, the Ac-AMPs have much higher specific activities. The nettle lectin only inhibits 6 out of 11 fungi at concentrations below 100 µg/ml, whereas at this concentration the pea chitinase is only inhibitory to 1 out of 7 tested fungi.

The unique properties of the Ac-AMPs as potent inhibitors of fungal growth in vitro suggest that they may play a role in the defence of seeds or seedlings against invasion by fungal organisms.

### EXAMPLE 9

### Effect of ions on antifungal activity.

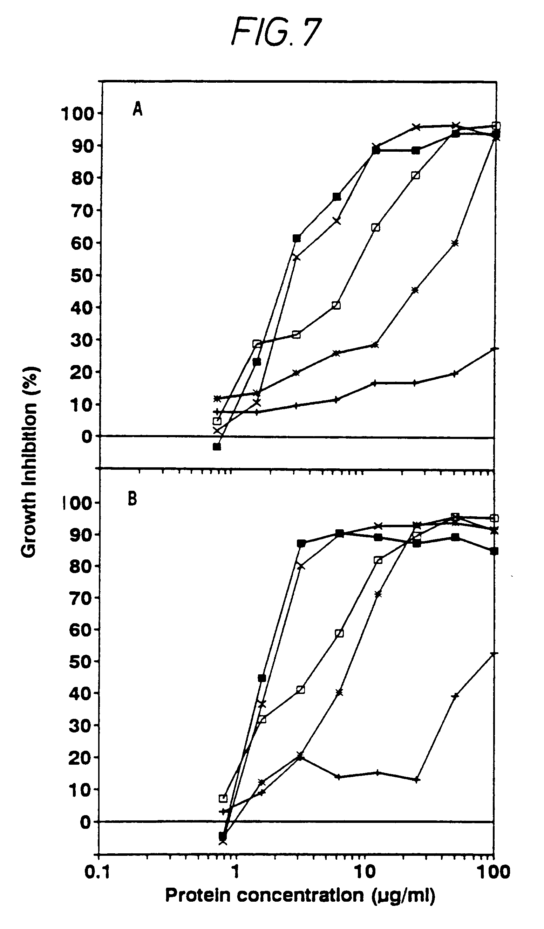

The specific activity of the Ac-AMPs was found to be strongly dependent on the ionic constitution of the growth medium. Figure 7 shows the dose-response curves of Ac-AMP1 (panel A) and Ac-AMP2 (panel B) on B cinerea in a low ionic strength synthetic growth medium, with and without different additions of KCl or CaCl₂. The antagonistic effect of K⁺ and Ca²⁺ on growth inhibition of B cinerea caused by the Ac-AMPs is obvious. In the reference medium (■), containing 2.5 mM monovalent cations and 0.1 mM divalent cations, Ac-AMP1 and Ac-AMP2 had IC₅₀ values of 2.2 and 1.6 µg/ml respectively. Administering KCl at 10 mM (x) to this medium did not significantly affect the dose-response curves, whereas KCl at concentrations of 50 mM (□) increased the IC₅₀ values by about three-fold. CaCl₂ had a much more dramatic antagonistic effect. When supplemented at 1 mM (*) to the reference medium, CaCl₂ caused a five to six-fold increase of the IC₅₀ values. At 5 mM CaCl₂ (+) the drops in specific activity were more than 50-fold. The antagonistic effect of other salts with monovalent cations, such as NaCl and NH₄Cl, was similar to that of KCl, whereas the effect of the salts with divalent cations, MgCl₂ and BaCl₂, was similar to that of CaCl₂.

These results show that the antifungal activity of the Ac-AMPs is strongly reduced by the presence of inorganic salts. The antagonistic effect of salts is primarily due to the cations; divalent cations are more potent antagonists than monovalent cations.

### EXAMPLE 10

### Effect of Ac-AMPs on bacteria.

Antibacterial activity was measured as described in Example 2. The following bacterial strains were used: Bacillus megaterium ATCC 13632, Erwinia carotovora strain 3912, Escherichia coli strain HB101 and Sarcina lutea ATCC 9342. The antibacterial effect of the Ac-AMPs was assessed by adding serial dilutions of the proteins to bacterial suspensions. The highest test concentration was 500 µg/ml (final concentration). Results are shown in Table 2.

**TABLE 2**

| Antibacterial activity of the Ac-AMPs | | |
|---|---|---|
| **Bacteria** | **IC**_{**50**} (µg/ml) | |
| | **Ac-AMP1** | **Ac-AMP2** |
| B megaterium | 40 | 10 |
| S lutea | 250 | 40 |
| E carotovora | no inhibition | |
| E coli | no inhibition | |

The Ac-AMPs inhibited growth of the gram-positive bacteria, B megaterium and S lutea. However, the Ac-AMPs (at 500 µg/ml) did not inhibit growth of the gram-negative bacteria E carotovora and E coli.

### EXAMPLE 11

### Effect of the purified antimicrobial proteins on cultured human cells.

The Ac-AMPs were evaluated for their potential toxic effects on mammalian cells.

Human cell toxicity assays were performed either on umbilical vein endothelial cells (Alessi et al, 1988, Eur J Biochem, 175, 531-540) or skin-muscle fibroblasts (Van Damme et al, 1987, Eur J Immunol, 17, 1-7) cultured in 96-well microplates. The growth medium was replaced by 80µl of serum-free medium (Optimem 1 for endothelial cells or Eagle's minimal essential medium (EMEM) for fibroblasts, both from GIBCO), to which 20 µl of a filter-sterilised test solution was added. The cells were further incubated for 24 hours at 37°C under a 5% CO₂ atmosphere with 100 % relative humidity. The viability of the cells was assessed microscopically after staining with trypane blue (400 mg/l in phosphate buffered saline, PBS) for 10 minutes. Alternatively, cells were stained with neutral red (56 mg/l in PBS) for 2 h at 37°C. Cells were lysed in acidic ethanol (100 mM sodium citrate, pH 4, containing 50% ethanol) and scored for release of the dye by microspectrophotometry at 540 nm.

When added at up to 500 µg/ml to either cultured human umbilical vein endothelial cells or human skin-muscle fibroblasts, neither Ac-AMP1 nor Ac-AMP2 affected cell viability after 24 h of incubation. In contrast, β-purothionin administered at 50 µg/ml decreased the viability of both cell types by more than 90%.

### EXAMPLE 12

### Molecular cloning of Ac-AMP2 cDNA.

Fully matured seeds of Amaranthus caudatus were collected from outdoor grown plants, immediately frozen in liquid nitrogen and stored at - 80°C. Total RNA was extracted from 5 g of pulverised seeds by the method of De Vries et al (1988, Plant Molecular Biology Manual, B6, 1-13) using 6 ml of a 1:2 phenol:RNA extraction buffer mixture and 2 ml of chloroform per g tissue. Poly (A)⁺ RNA was purified by oligo (dT)-cellulose affinity chromatography as described by Silflow et al (1979, Biochemistry, 18, 2725-2731) yielding about 7 µg of poly (A)⁺ RNA. Double-stranded cDNAs were prepared from 1.5 µg of poly (A)⁺ RNA according to Gubler and Hoffman (1983, Gene, 25, 263-269) and ligated to EcoRI/NotI adaptors using the cDNA Synthesis Kit of Pharmacia. The cDNAs were cloned into the λ ZAP II phage vector (Stratagene) and packaged in vitro with the Gigapack II Gold packaging system (Stratagene) according to the manufacturer's instructions.

A DNA probe for screening of the cDNA library was produced by polymerase chain reaction (PCR) as follows. Two degenerate oligonucleotides were synthesised: OWB13 (5'GTNGGNGARTGKGTNMGNGG) and OWB14 (5'CCRCARTAYTTNGGNCCYTTNCC). OWB13 corresponds to amino acids 1 to 7 of Ac-AMP1 and has a sense orientation. OWB14 corresponds to amino acids 22 to 29 of Ac-AMP1 and has an antisense orientation. PCR was performed with the Taq polymerase under standard conditions (Sambrook et al, 1989, Molecular Cloning, Cold Spring Harbour Lab Press) using OWB13 and OWB14 as amplimers and 25ng of cDNA as target DNA. The temperature programme included an initial step at 94°C for 5 min, 30 cycles (94°C for 1 min; 45°C for 2 min; 72°C for 3 min) and a final step at 72°C for 10 min. The 100 bp PCR amplification product was purified on a 3% agarose (NuSieve, FMC) gel and reamplified by PCR under the same conditions except that the reaction mixtures contained 130 µM dTTP and 70 µM digoxigenin-11-dUTP instead of 200 µM dTTP. The digoxigenin-labelled PCR product was purified on a 3% NuSieve agarose gel.

About 100,000 plaque forming units of the λ ZAP II cDNA library were screened with the digoxigenin-labelled PCR product by in situ plaque hybridisation using nylon membranes (Hybond -N, Amersham). Membranes were air-dried and DNA was crosslinked on the membranes under UV light (0.15 J/cm²). Hybridisation was performed for 16 hours at 65°C in 5 x SSC, 1% blocking reagent (Boehringer Mannheim), 0.1% N-lauroylsarcosine, 0.02% sodium dodecylsulphate containing 10 ng/ml of heat denatured digoxigenin-labelled probe.
Non-specifically bound probe was removed by rinsing twice for 5 min in 2 x SSC/0.1% SDS at 25°C and twice for 15 min in 0.1 x SSC/0.1% SDS at 60°C. Detection of the probe was done using anti-digoxigenin antibodies linked to alkaline phosphatase (Boehringer Mannheim) and its substrate 5-bromo-4-chloro-3-indolyl phosphate (Boehringer Mannheim) according to the manufacturer's instructions. Positive plaques were purified by two additional screening rounds with the same probe under the same conditions. Inserts from purified plaques were excised in vivo into the pBluescript phagemid form with the aid of the helper phage R408, according to the instructions of Stratagene. Nucleotide sequencing was done with an ALF automated sequencer (Pharmacia) using fluoresceine-labelled M13 forward and reverse primers (Pharmacia). Sequence analysis was performed by the Intelligenetics PC-gene software.

Inserts from ten different positive clones were released by EcoRI digestion and their sizes compared by agarose electrophoresis. The clone with the longest insert (AC1) was subjected to nucleotide sequence analysis. AC1 is 590 nucleotides long and contains an open reading frame of 86 amino acids. The 25 amino-terminal amino acids have a predictable signal peptide structure obeying the (-1,-3)-rule (Von Heijne, 1985, Mol Biol, 184, 99-105). The deduced amino acid sequence of the region following the putative signal peptide is identical to the 30-amino acid sequence of mature Ac-AMP2 as determined by protein sequencing. In addition, the mature protein domain is extended by a 31-amino acids carboxy-terminal domain. This carboxy-terminal extension may play a role in the subcellular targeting of Ac-AMP2. AC1 has 45-nucleotide and 284-nucleotide untranslated regions at the 5' and 3' end, respectively. The 3' end untranslated region is not terminated by a poly(A) tail, indicating that AC1 is not a full length cDNA clone.

All of the other nine sequenced positive clones had deduced amino acid sequences identical to that of AC1. They differed from each other by various degrees of truncation at the 5' or 3' end. The fact that the 10 sequenced clones all contained an arginine at position 30 of the mature protein domain suggests that Ac-AMP1 and Ac-AMP2 are both derived from the same precursor preprotein.

The carboxy-terminal extension peptide did not show relevant homology either at the amino acid or nucleotide level with any of the entries from the Swiss-Prot (release 20) or EMBL gene bank (release 29), respectively. The structure of the Ac-AMP2 cDNA thus appears to be unique and obviously different from that of known genes encoding chitin-binding proteins.

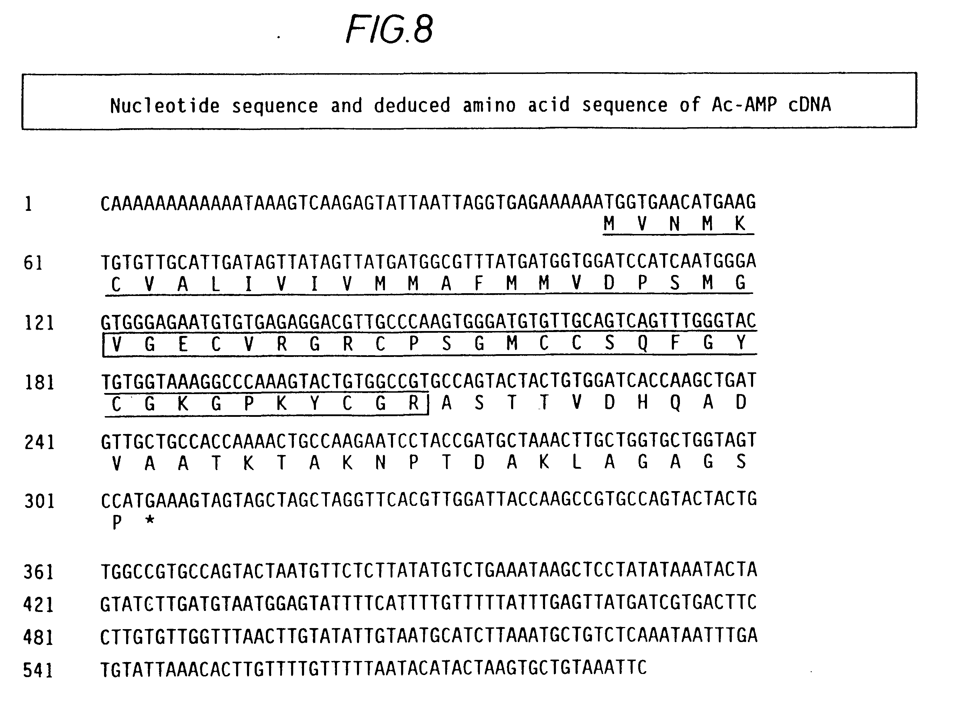

Figure 8 shows the nucleotide sequence and deduced amino acid sequence of clone AC1. The putative signal sequence is underlined and the sequence of mature Ac-AMP2 is boxed. The stop codon is marked with an asterisk.

### EXAMPLE 13

### Construction of the expression vectors pAC11 and pAC12.

Two different expression cassettes were constructed based on two portions of the insert of clone AC1.

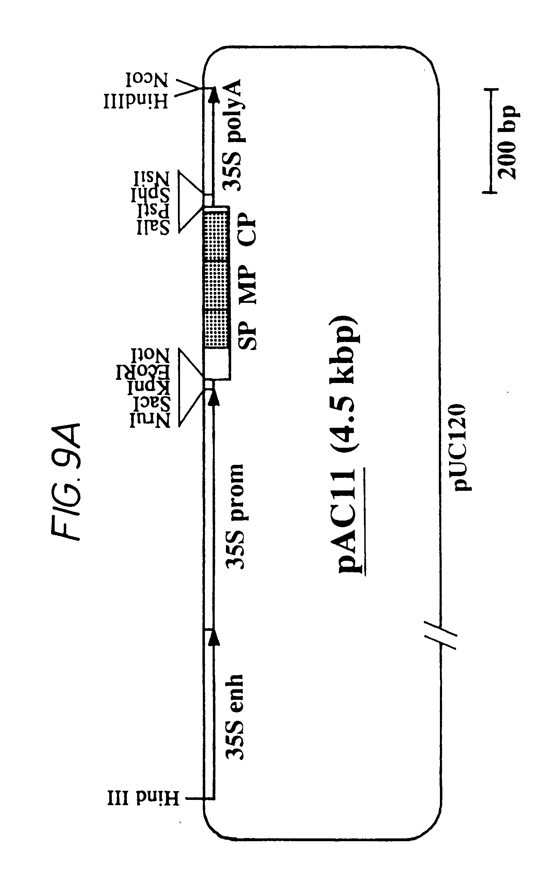

The first expression vector (pAC11, as shown in Figure 9A) contains the full coding region of Ac-AMP2 cDNA flanked at its 5' end by the strong constitutive promoter of the 35S RNA of cauliflower mosaic virus, CaMV35S (Odell et al, 1985, Nature, 313, 810-812) with a duplicated enhancer element to allow for high transcriptional activity (Kay et al, 1987, Science, 236, 1299-1302). The coding region of Ac-AMP2 cDNA is flanked at its 3' side by the CaMV35S polyadenylation sequence. The plasmid backbone of this vector is the phagemid pUC120 (Vieira and Messing, 1987, Methods Enzymol, 153, 3-11).

pAC11 was constructed from clone AC1 as follows. Clone AC1 consists of the Ac-AMP2 cDNA (shown in Figure 8) cloned into the EcoRI site of pBluescript SK(+) (from Stratagene) such that the 5' end faces the M13 universal primer binding site. AC1 was digested with EcoRV (cuts within the SK polylinker of pBluescript) and NheI (cuts internally in the Ac-AMP2 cDNA sequence at base position 315 which is 9 bases downstream of the stop codon). The EcoRV/NheI 332 bp fragment was subcloned into the expression vector pFAJ3002 which was pre-digested with SmaI and XbaI. pFAJ3002 is a derivative of the expression vector pFF19 (Timmermans et al, 1990, J Biotechnology, 14, 333-344) in which the unique EcoRI site is replaced by a HindIII site.

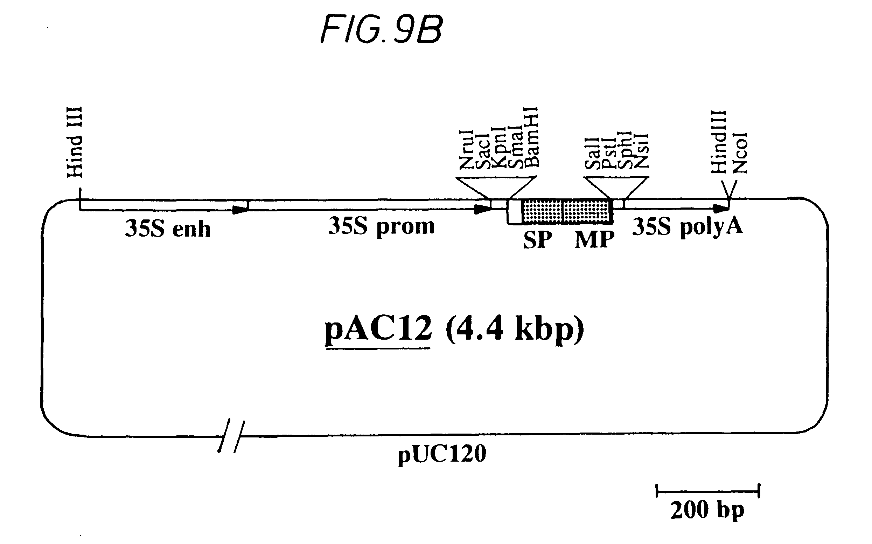

The second expression vector (pAC12, shown in Figure 9B) contains an open reading frame coding for the signal peptide and mature domain of the Ac-AMP preprotein. This open reading frame is flanked at its 5' side by the duplicated CaMV35S promoter and at its 3' side by the CaMV35S polyadenlyation sequence.

pAC12 was constructed as follows. A 216 bp fragment was amplified by polymerase chain reaction using AC1 as DNA template and OWB32, OWB33 as sense and antisense primers respectively. The primer OWB32 (5'AATTGGATCCAGTCAAGAGTATTAATTAGG) corresponds to nucleotides 17 to 36 of Ac-AMP2 cDNA and introduces a BamHI site at the 5' end of the PCR amplification product. The primer OWB33 (5'AATTGTCGACTCAACGGCCACAGTACTTTGGGCC) corresponds to nucleotides 190 to 210 of Ac-AMP2 cDNA and links an inframe stop codon and a SalI site to the 3' end of the PCR products. Both OWB32 and OWB33 have a 4 bp random sequence at the 5' end prior to the restriction site. The PCR product was digested with BamHI and SalI and subsequently subcloned into the expression vector pFAJ3002, previously digested with the same restriction enzymes.

### EXAMPLE 14

### Construction of the plant transformation vectors pAC111 and pAC112.

The expression vector pAC11 and pAC12 were digested with HindIII and the fragments containing the Ac-AMP2 cDNA expression cassettes were subcloned into the unique HindIII site of pBinl9Ri. pBinl9Ri is a modified version of the plant transformation vector pBinl9 (Bevan, 1984, Nucleic Acids Research, 12:22, 8711-8721) wherein the unique EcoR1 and HindIII sites are switched and the defective npt II expression cassette (Yenofsky et al, 1990, Proc Natl Acad Sci USA, 87:3435-3439) is replaced by the npt II expression cassette described by An et al (1985, EMBO J, 4:277-284).

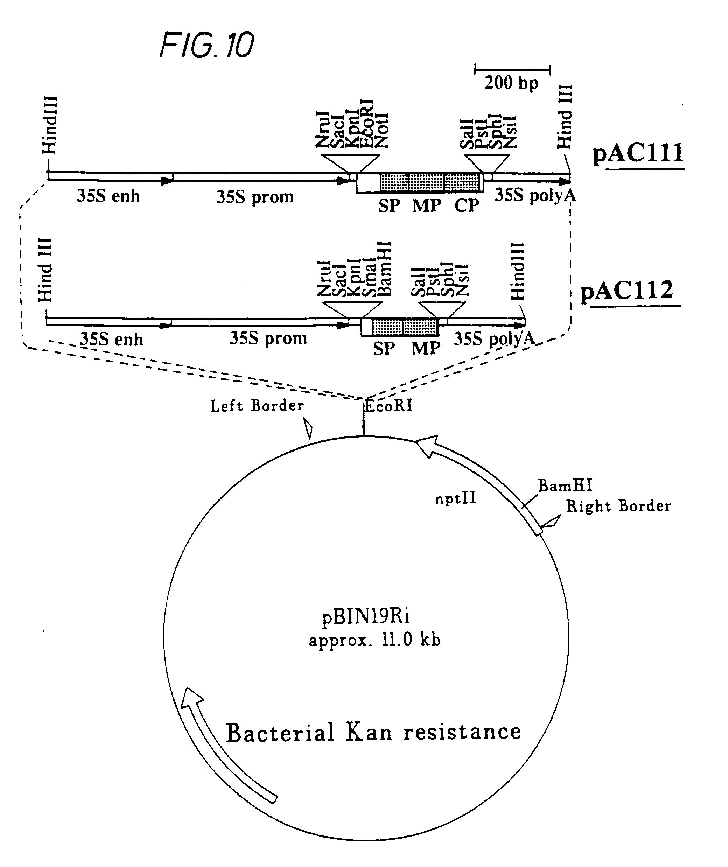

The plant transformation vector containing the pAC11 expression cassette was designated pAC111, while the transformation vector containing the pAC12 expression cassette was designated pAC112. The structure of the two transformation vectors is shown in Figure 10.

### EXAMPLE 15

### Plant Transformation.

The disarmed Agrobacterium tumefaciens strain LBA4404 [pAL4404] (Hoekema et al, 1983, Nature, 303:179-180) may be transformed by either of the vectors pAC111 or pAC112, using the method of de Framond A et al (Biotechnology, 1:262-9).

Tobacco transformation may be carried out using leaf discs of Nicotiana tabacum Samsun based on the method of Hozsch RB et al (1985, Science, 227, 1229-31) and co-culturing with Agrobacterium strains containing pAC111 or pAC112.
Co-cultivation may be carried out under selection pressure of 100 µg/ml kanamycin. Transgenic plants (transformed with pAC111 or pAC112) may be regenerated on media containing 100 µg/ml kanamycin. These transgenic plants may be analysed for expression of the newly introduced genes using standard Western blotting techniques. Transgenic plants may also be analysed for increased resistance to fungal or bacterial diseases.

Plants capable of constitutive expression of the introduced genes may be selected and self-pollinated to give seed. The progeny of the seed exhibiting stable integration of the Ac-AMP genes would be expected to show typical Mendelian inheritance patterns for the Ac-AMP genes.

## Claims

1. An isolated anti-microbial protein which is a member of a class of proteins having an amino acid sequence containing the cysteine/glycine domain of chitin-binding proteins derived from plants **characterised in that** said anti-microbial protein is capable of being isolated from *Amaranthus* seeds or seeds of a closely related species and has:
(a) a higher ratio of basic amino acids to acidic amino acids; and
(b) an additional amino-terminal residue; and
(c) the occurrence of a gap of four amino acids at position 6 to 9; and
(d) the lack of a carboxyl terminal portion of 10-12 residues
when compared to the regular 40-43 amino acid cysteine/glycine-rich domain found in said chitin-binding proteins.

2. The pure protein Ac-AMP1 or Ac-AMP2.

3. A recombinant DNA sequence coding for a protein as claimed in claim 1 or claim 2.

4. A vector containing a DNA sequence according to claim 3.

5. Plants transformed with recombinant DNA according to claim 3 or a vector according to claim 4.

6. Plants according to claim 5 wherein said DNA encodes the protein Ac-AMP1 or Ac-AMP2.

7. An anti-microbial composition containing one or more of the proteins as claimed in claim 1 or claim 2.

8. A biological system including a DNA according to claim 3 which allows expression of the encoded protein wherein said system is a plant, a micro-organism, a cultured insect cell or a cultured mammalian cell.

9. A process of combating fungi or bacteria which comprises exposing fungi or bacteria to a protein according to claim 1 or 2 or a composition according to claim 7 wherein the process is not an *in vivo* one.

10. An extraction process for producing a protein as claimed in claim 1 or claim 2 from organic material containing said protein which process comprises submitting the organic material to maceration and solvent extraction, and optionally purifying the protein by centrifugation, chromatography and dialysis.

## Patentansprüche

1. Isoliertes antimikrobielles Protein, das ein Glied einer Klasse von Proteinen mit einer Aminosäuresequenz ist, die die Cystein/Glycin-Domäne von aus Pflanzen stammenden Chitin-Bindungsproteinen enthält, **dadurch gekennzeichnet, daß** das antimikrobielle Protein aus *Amaranthus-Samen* oder aus Samen eng verwandter Spezies isolierte werden kann und folgendes aufweist:
(a) ein höheres Verhältnis von basischen Aminosäuren zu sauren Aminosäuren; und
(b) einen zusätzlichen aminoterminalen Rest; und
(c) das Auftreten einer Lücke von vier Aminosäuren in Position 6 bis 9; und
(d) das Fehlen eines carboxyterminalen Teils von 10-12 Resten
im Vergleich zu der regulären, in den Chitin-Bindungsproteinen vorkommenden Cystein/Glycin-reichen 40-43-Aminosäure-Domäne.

2. Reines Ac-AMP1- oder Ac-AMP2-Protein.

3. Rekombinante DNA-Sequenz, die ein Protein nach Anspruch 1 oder Anspruch 2 codiert.

4. Vektor, der eine DNA-Sequenz nach Anspruch 3 enthält.

5. Pflanzen, die mit rekombinanter DNA nach Anspruch 3 oder einem Vektor nach Anspruch 4 transformiert worden sind.

6. Pflanzen nach Anspruch 5, wobei die DNA das Protein Ac-AMP1 oder Ac-AMP2 codiert.

7. Antimikrobielle Zusamensetzung, die ein oder mehrere Proteine nach Anspruch 1 oder Anspruch 2 enthält.

8. Biologisches System, einschließlich einer DNA nach Anspruch 3, die die Expression des codierten Proteins gestattet, wobei das System eine Pflanze, ein Mikroorganismus, eine gezüchtete Insekten- oder Säugerzelle ist.

9. Verfahren zur Bekämpfung von Pilzen oder Bakterien, das die Exposition von Pilzen oder Bakterien gegenüber einem Protein nach Anspruch 1 oder 2 oder einer Zusammensetzung nach Anspruch 7 umfaßt, wobei das Verfahren kein *in*-*vivo*-Verfahren ist.

10. Extraktionsverfahren zur Herstellung eines Proteins nach Anspruch 1 oder Anspruch 2 aus einem organischen Material, das das Protein enthält, wobei das Verfahren die Durchführung einer Mazeration und Lösungsmittelextraktion mit dem organischen Material und gegebenenfalls die Reinigung des Proteins durch Zentrifugation, Chromatographie und Dialyse umfaßt.

## Revendications

1. Protéine antimicrobienne isolée qui est un élément d'une classe de protéines ayant une séquence d'aminoacides contenant le domaine cystéine/glycine des protéines fixant la chitine, dérivées de plantes, **caractérisée en ce que** ladite protéine antimicrobienne est capable d'être isolée à partir de graines d'*Amaranthus* ou de graines d'une espèce étroitement apparentée et a :
(a) un rapport supérieur des aminoacides basiques aux aminoacides acides ; et
(b) un résidu amino-terminal supplémentaire ; et
(c) l'apparition d'une brèche de quatre aminoacides aux positions 6 à 9 ; et
(d) l'absence d'une partie terminale carboxyle de 10-12 résidus,
par comparaison au domaine habituel de 40-43 aminoacides riche en cystéine/glycine trouvé dans lesdites protéines fixant la chitine.

2. Protéine pure Ac-AMP1 ou Ac-AMP2.

3. Séquence d'ADN recombinant codant pour une protéine selon la revendication 1 ou la revendication 2.

4. Vecteur contenant une séquence d'ADN selon la revendication 3.

5. Plantes transformées avec un ADN recombinant selon la revendication 3 ou un vecteur selon la revendication 4.

6. Plantes selon la revendication 5, dans lesquelles ledit ADN code pour la protéine Ac-AMP1 ou Ac-AMP2.

7. Composition antimicrobienne contenant une ou plusieurs des protéines selon la revendication 1 ou la revendication 2.

8. Système biologique comprenant un ADN selon la revendication 3, qui permet l'expression de la protéine codée, ledit système étant une plante, un micro-organisme, une cellule d'insecte mise en culture ou une cellule mammifère mise en culture.

9. Procédé pour lutter contre des champignons ou des bactéries, comprenant l'exposition des champignons ou des bactéries à une protéine selon la revendication 1 ou 2 ou à une composition selon la revendication 7, ledit procédé n'étant pas un procédé *in vivo.*

10. Procédé d'extraction pour produire une protéine selon la revendication 1 ou la revendication 2 à partir d'une matière organique contenant ladite protéine, ledit procédé comprenant les étapes consistant à soumettre la matière organique à une macération et à une extraction avec un solvant, et éventuellement à purifier la protéine par centrifugation, chromatographie et dialyse.
